(19) Europäisches Patentamt / European Patent Office / Office européen des brevets

(11) **EP 3 477 278 B1**

(12) **EUROPÄISCHE PATENTSCHRIFT**

(45) Veröffentlichungstag und Bekanntmachung des Hinweises auf die Patenterteilung:
**22.04.2020   Patentblatt 2020/17**

(51) Int Cl.:
**G01N 21/17** *(2006.01)*    **A61B 5/145** *(2006.01)*
**A61B 5/00** *(2006.01)*

(21) Anmeldenummer: **17198860.3**

(22) Anmeldetag: **27.10.2017**

(54) **PHOTOAKUSTIK-VERFAHREN MIT EINEM MESSLICHT AUFWEISEND EINEN VORBESTIMMTEN WELLENLÄNGENBEREICH ZUR BESTIMMUNG VON EIGENSCHAFTEN EINER INHOMOGENEN PROBE**

PHOTOACOUSTICS METHOD WITH A MEASURING LIGHT COMPRISING A PREDETERMINED WAVELENGTH RANGE FOR DETERMINING THE PROPERTIES OF AN INHOMOGENEOUS SAMPLE

PROCÉDÉ DE PHOTOACOUSTIQUE À UNE LUMIÈRE DE MESURE COMPRENANT UNE PLAGE DE LONGUEURS D'ONDE PRÉDÉTERMINÉE PERMETTANT LA DÉTERMINATION DES PROPRIÉTÉS D'UN ÉCHANTILLON INHOMOGÈNE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**01.05.2019   Patentblatt 2019/18**

(73) Patentinhaber: **Humboldt-Universität zu Berlin**
**10099 Berlin (DE)**

(72) Erfinder:
• **Schlesinger, Raphael**
**12165 Berlin (DE)**
• **Kischkat, Jan-Ferenc**
**10829 Berlin (DE)**

• **von Lilienfeld-Toal, Hermann**
**63571 Gelnhausen (DE)**

(74) Vertreter: **RCD Patent**
**Giesen, Schmelcher & Griebel**
**Patentwanwälte PartG mbB**
**Kaiserstraße 100**
**52134 Herzogenrath (DE)**

(56) Entgegenhaltungen:
**US-A1- 2010 070 233**

• **FEI GAO ET AL: "Single laser pulse generates dual photoacoustic signals for differential contrast photoacoustic imaging", SCIENTIFIC REPORTS, Bd. 7, Nr. 1, 4. April 2017 (2017-04-04), XP55433123, DOI: 10.1038/s41598-017-00725-4**

EP 3 477 278 B1

# EP 3 477 278 B1

**Beschreibung**

Die Erfinndung betrifft ein Photoakustik-Verfahren zur Bestimmung von Eigenschaften einer inhomogenen Probe mit Mikrometer-Tiefenauflösung. Insbesondere betrifft die Erfindung ein Photoakustik-Verfahren, bei dem elektromagnetische Strahlung auf eine Oberfläche einer Probe eingestrahlt und von der Probe unter Erzeugen einer Druckwelle absorbiert wird, wobei die Druckwelle zur Messfläche propagiert und als eine Drucktransiente detektiert wird.

Die Erfindung betrifft auch ein Photoakustik-Verfahren zur nichtinvasiven Bestimmung von Eigenschaften lebenden Gewebes, z.B. lebender Haut.

Photoakustik-Messapparate und -Sensoren sind bekannte Mittel zur Detektion und Quantifizierung von Substanzen durch deren charakteristische Lichtabsorption. Zur Untersuchung einer Probe auf eine gesuchte Substanz erfolgt die Beleuchtung der Probe mit einem Messlicht, das eine oder mehrere Lichtwellenlängen aufweist, die als charakteristische Absorptionswellenlängen der Substanz vorbekannt sind. Das Messlicht wird gepulst in die Probe eingestrahlt und dort in Abhängigkeit von der Verteilung der Substanz lokal unterschiedlich stark absorbiert. Die mit der Lichtabsorption eingetragene Energie bewirkt eine Erwärmung und eine thermomechanische Expansion der Probe. Beides löst Relaxationsprozesse aus, die die inhomogen eingetragene Energie über die Probe verteilen, um das Equilibrium wieder herzustellen. Dies geschieht über Wärmediffusion und die Propagation von Druckwellen - Schallwellen - durch die Probe, u.a. hin zur Probenoberfläche.

Bei gängigen Photoakustik-Verfahren werden die Druckwellen als Drucktransienten an der Probenoberfläche von piezoelektrischen Schallwandlern detektiert. Solche Schallwandler, i. F. auch Detektoren, können mit einer entsprechenden Messelektronik hohe Zeitauflösungen von üblich einigen zehn Nanosekunden erreichen und eignen sich daher zur Unterscheidung von Druckwellenlaufzeiten aus verschiedene Tiefen der Probe. Wählt man die Wellenlänge des Messlichts so aus, dass es vorwiegend von lokalisierten Substanzen oder Strukturen (z.B. Hämoglobin in Blutgefäßen) absorbiert wird und detektiert man die Drucktransienten an einer Mehrzahl von Orten auf der Proben simultan, dann kann man die Orte der Absorption ermitteln und als 3D-Bild darstellen. So lassen sich beispielsweise Darstellungen von Blutgefäßnetzen in vivo erzeugen. Aufgrund der großen Schallgeschwindigkeit in festen oder flüssigen Proben, z.B. 1,5 $\mu$m/ns in Wasser, ist herkömmliche Photoakustik aber nur begrenzt zur Untersuchung einer Probe geeignet, wenn diese mit einem Messlicht bestrahlt wird, das die Probe bereits in einer Tiefe von einigen zehn Mikrometern unter der Probenoberfläche vollständig absorbiert hat. Unter diesen Bedingungen sollte das Drucksignal auf derselben Messfläche in der Oberfläche detektiert werden, in die das Messlicht zuvor eingestrahlt worden ist, da praktisch nur direkt unter der Messfläche Absorption stattfinden kann. Entsprechend kurz sind alle Laufzeiten der annähernd gleichzeitig am Detektor eintreffenden Drucksignale. Die begrenzte Zeitauflösung des Detektors erlaubt in der Regel keine Mikrometer-Auflösung für die Probentiefen, aus denen die Drucksignale stammen.

In der Veröffentlichung von Fei Gao et al., "Single laser pulse generated dual photoacoustic signals for differential contrast photoacoustic imaging", Scientific Reports | 7: 626 | DOI:10.1038/s41598-017-00725-4 1, (2017) wird die Verwendung langer Messlichtpulse vorgeschlagen, um mit jedem Puls zwei zeitlich separierte Drucksignale auszulösen, nämlich je eines am Anfang und am Ende eines Messlichtpulses. Die beiden zu einem Messlichtpuls gehörenden Drucksignale sind dabei nicht-linear korreliert. Sie weisen einen festen Zeitabstand und entgegengesetzte Polarität auf, können aber unterschiedliche Amplituden besitzen in Abhängigkeit von der Messlichtpulsdauer und von der Erwärmung der Probe während der langen Pulsdauer. Aus der Differenz der korrelierten Drucksignale lassen sich insofern Erkenntnisse über die in der Probe deponierte Energiedichte gewinnen. Das Verfahren von Fei Gao et al. soll im Folgenden als Differentielle Photoakustik (DPA) bezeichnet werden.

Beispielsweise aus dem Paper von Kottmann et al., "Glucose sensing in human epidermis using midinfrared photoacoustic detection", Vol. 3, No. 4 / BIOMEDICAL OPTICS EXPRESS, S. 667 ff., geht die folgende Messaufgabe hervor: das Bestimmen des Blutzuckergehalts in vivo durch Messen der Absorption von MIR-Messlicht mit einer Wellenlänge um 10 $\mu$m im "interstitial fluid" (ISF) unterhalb der etwa 20 $\mu$m dicken Stratum Corneum (SC) der lebenden Haut. Das Messlicht wird dabei laut Quelle innerhalb eines Tiefenbereichs von bis zu 100 $\mu$m unter der Hautoberfläche absorbiert, und die Absorption generiert ein Drucksignal. Das SC selbst liefert aber keine aussagekräftige Information über die Blutzuckerkonzentration. Erst die Absorption in tiefer gelegenen Schichten, insbesondere im Stratum Spinosum, ist dazu geeignet, den Glukoselevel abzuleiten. Solche variierenden Eigenschaften einer inhomogenen Probe zu ermitteln, setzt das Erreichen einer Mikrometer-Tiefenauflösung auf lediglich wenigen zehn Mikrometern Messtiefe direkt unter der Oberfläche voraus.

Vor diesem Hintergrund besteht ein Interesse an einem neuen photoakustischen Messverfahren, das inhomogene Proben mit Mikrometer-Tiefenauflösung untersuchen kann, gerade dann, wenn das Messlicht aus einem Spektralbereich stammt, der von der inhomogenen Probe stark absorbiert wird. Nach Kenntnis der Erfinder wurde die Differentielle Photoakustik für diese Fragestellung noch nicht als eine Lösungsmöglichkeit in Betracht gezogen.

Die Erfindung stellt sich die Aufgabe, ein Photoakustik-Verfahren mit einem Messlicht aufweisend einen vorbestimmten Wellenlängenbereich zur Bestimmung von Eigenschaften einer inhomogenen Probe vorzuschlagen, wobei die Probe für den vorbestimmten Wellenlängenbereich eine mittlere Absorptionslänge aus dem Intervall 1 - 100 Mikrometer aufweist.

Die Aufgabe wird gelöst durch ein Photoakustik-Verfahren mit einem Messlicht aufweisend einen vorbestimmten Wellenlängenbereich zur Bestimmung von Eigenschaften einer inhomogenen Probe, wobei die Probe für den vorbestimmten Wellenlängenbereich eine mittlere Absorptionslänge $\mu$ aus dem Intervall 1 - 100 Mikrometer aufweist, umfassend die Schritte:

a) Einstrahlen von wenigstens einem Messlichtpuls mit vorbestimmter Pulsdauer und vorbestimmter Intensität auf eine Messfläche der Fläche $F$ mit $\sqrt{F} \gg \mu$ in der Oberfläche der inhomogenen Probe;

b) Detektieren von wenigstens einer Drucktransienten an der Messfläche, wobei die Drucktransiente durch Absorption des wenigstens einen Messlichtpulses in der inhomogenen Probe unter Erzeugen von einer zur Messfläche propagierenden Druckwelle entsteht;

c) Berechnen eines Wertes für die während der Pulsdauer von der Probe absorbierte Energiedichte aus dem Verlauf der wenigstens einen Drucktransienten am Anfang und am Ende des wenigstens einen Messlichtpulses; gekennzeichnet durch

d) Wiederholen der Schritte a) bis c) für voneinander verschiedene Einfallswinkel des Messlichts gegenüber der Normalen der Messfläche, wobei die in c) bestimmten Energiedichtewerte jeweils mit dem Einfallswinkel indiziert werden;

e) Modellieren der inhomogenen Probe als Schichtenstapel, wobei jeder Schicht wenigstens eine Schichtdicke und ein Absorptionskoeffizient zugeordnet werden, wobei wenigstens ein Absorptionskoeffizient einer Schicht ein Fitparameter ist;

f) Ausführen einer Fitprozedur für die Fitparameter des Schichtenstapels, wobei durch Variation der Fitparameter die Aufteilung der mit dem Einfallswinkel indizierten Energiedichtewerte auf Beiträge der einzelnen Schichten variiert wird, bis ein vorbestimmtes Konsistenzkriterium erfüllt ist;

g) Auslesen der gefitteten Fitparameter als Werte wenigstens für den tiefenaufgelösten Absorptionskoeffizienten der inhomogenen Probe.

Die Unteransprüche geben vorteilhafte Ausgestaltungen des Verfahrens an.

Das erfindungsgemäße Photoakustik-Verfahren analysiert den zeitlichen Verlauf von detektierten Drucktransienten, verzichtet dabei aber auf jegliche Zuordnung von Schalllaufzeiten auf Probentiefen. Vielmehr werden prominente Strukturen der Drucktransienten, die sich am Anfang und am Ende eines Messlichtpulses zeigen und hier als Druckpulse bezeichnet werden, zeitlich voneinander separiert erfasst und beispielsweise und bevorzugt zeitlich integriert. Die Differenz der Druckpulsintegrale, $\Delta p$, einer einzelnen Drucktransiente ist das Resultat einer unterschiedlichen Expansionsantwort auf das Messlicht am Anfang und am Ende des langen Messlichtpulses. Beispielsweise erfolgt in der Regel eine Expansion der Probe, wenn der Messlichtpuls in der Probe eintrifft, und es erfolgt eine Kontraktion, sobald der Messlichtpuls endet, d.h. die Beleuchtung abgeschaltet wird. Während der Dauer des Messlichtpulses wird elektromagnetische Energie in der Probe deponiert, was zur lokalen Erwärmung führt. Der temperaturabhängige Grüneisen-Koeffizient, $\Gamma(T)$, des beleuchteten Volumens der Probe ist ein bestimmender Parameter für die photoakustische Antwort und in erster Näherung linear von der Temperaturänderung abhängig:

$$\Gamma(T) = \Gamma_0 + \Gamma'\Delta T \tag{1}$$

Zugleich wird die Temperaturerhöhung im beleuchteten Volumen infolge der absorbierten Strahlung durch die Wärmekapazität $C_p$ der Probe bestimmt. Sind diese Materialkoeffizienten für die untersuchte Probe bekannt oder kann man darüber aus der Kenntnis der Probe heraus vernünftige Annahmen machen, dann lässt sich die unmittelbare Messgröße $\Delta p$ in die deponierte Energie, $\Delta E$, bezogen auf das beleuchtete Volumen, $V$, d.h. in die absorbierte Energiedichte, umrechnen:

$$\Delta p = \eta I_0 \delta t \times \frac{\Gamma'}{C_p} \times \frac{\Delta E}{V} \tag{2}$$

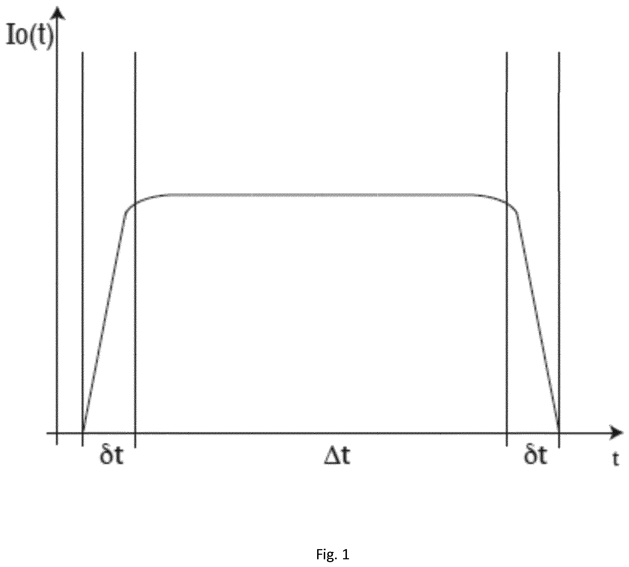

In Gleichung (2) bezeichnet $I_0$ die auf die Probe eingestrahlte Messlichtintensität (in Einheiten Leistung/Fläche), $\delta t$ die Anstiegs- oder Abklingdauer des Messlichtpulses (vgl. auch Fig. 1) und $\eta$ die Umwandlungseffizienz von Wärme in Druck in Analogie zu Fei Gao et al. (2017), geteilt durch die Schallgeschwindigkeit und die detektorabhängige Integrationszeit des Drucktransienten.

Die Grundidee der Erfindung basiert nun auf der Erkenntnis, dass $\Delta p$ eine deutliche Abhängigkeit vom Einfallswinkel, $\theta$, des Messlichts gegen die Normale der Messfläche zeigt, gerade weil das Messlicht gemäß Voraussetzung schon auf kurzen Strecken in der Probe stark absorbiert wird. Es erreicht bei großen Winkeln tiefer liegende Schichten der Probe nicht mehr, die es bei senkrechtem Einfall ($\theta = 0°$) noch erreichen kann. Somit repräsentiert eine Sequenz von Messungen von $\Delta p(\theta)$ zu voneinander verschiedenen - gleichbedeutend: jeweils paarweise verschiedenen - Winkeln $\theta$ einen Satz linear unabhängiger Messungen der Probe, aus dem sich auch eine Information über die Tiefenauflösung der Probe extrahieren lässt - und zwar ganz zwangsläufig dann auf der Mikrometerskala.

Das erfindungsgemäße Verfahren ähnelt etwas einer Tomographie, etwa der Röntgen-CT, bei der ein Objekt unter verschiedenen Winkeln durchstrahlt und abgebildet wird, und aus der Zusammenschau der gewonnenen Bilder hiernach ein Computer-Modell rekonstruiert wird.

Die Erfindung muss ebenfalls eine Rekonstruktion der inhomogenen Probe vornehmen, wobei diese Rekonstruktion die deutlich vereinfachende Annahme machen kann, dass Strukturänderungen nur entlang der Tiefenachse auftreten. Die inhomogene Probe kann dann als ein eindimensionaler Schichtenstapel umfassend eine Anzahl M Schichten modelliert werden, wobei M eine natürliche Zahl ist. Erfindungsgemäß werden jeder Schicht (mit Index $i$ = 1, ...,$M$) des Modells wenigstens eine Schichtdicke, $d_i$, und ein Absorptionskoeffizient, $\alpha_i$, als Eigenschaft zugewiesen, wobei wenigstens ein Absorptionskoeffizient einer Schicht als Fitparameter vorgesehen ist.

Die einzigen unmittelbar winkelabhängigen Größen auf der rechten Seite von Gleichung (2) sind die deponierte Energie $\Delta E(\theta)$ und das beleuchtete Volumen $V(\theta) = F \times \mu(\theta)$, wobei mit $F$ die Fläche der vorbestimmten Messfläche bezeichnet ist, auf die das Messlicht eingestrahlt wird, und mit $\mu(\theta)$ die über die Probe gemittelte Eindringtiefe der Strahlung. Tatsächlich erfolgt senkrecht zur Tiefenachse, also parallel zur Probenoberfläche, bereits eine implizite Mittelung der photoakustischen Antwort der Probe, weil die Abmessungen der Messfläche, z.B. Kantenlängen oder Durchmesser jeweils proportional zu $\sqrt{F}$, sehr groß gegenüber $\mu$ eingerichtet sind.

Dass der in Wahrheit beleuchtete Probenbereich aufgrund von Brechung des Messlichts bei schrägem Einfall auch noch einen Randbereich aufweisen kann, der nicht unter der Messfläche liegt, wird hier zur Vereinfachung vernachlässigt.

Die Verteilung der in der Probe deponierten Energiedichte hängt wesentlich davon ab, welche Weglängen das Messlicht in welchen Schichten - unter der Wirkung des jeweiligen Absorptionskoeffizienten - zurücklegt. Beispielsweise bei einem Einfallswinkel nahe 90° - streifender Einfall - ist es möglich, dass das Messlicht vollständig in der obersten Schicht der Probe propagiert und absorbiert wird. Wird der Winkel verringert, dann ändert sich die Eindringtiefe und das Messlicht kann auch in einer zweiten oder auch noch in einer dritten Schicht usw. absorbiert werden.

Die Veränderung des Einfallswinkels kommt effektiv der simultanen Streckung aller Schichtdicken des Modells der Probe gleich. Anders gesagt reicht es aus, für das Schichtmodell die Lichtintensität $I(x)$ mit x als Tiefenachse aus den Modellparametern zu berechnen und für die verschiedenen Winkel $\theta$ dabei einfach alle $d_i$ durch $d_i/\cos(\theta)$ zu ersetzen. Zur Verdeutlichung: Es ist für jede Variation der Schichtdicken $d_i$ oder des Winkels $\theta$ eine Neuberechnung der Intensität $I(x)$ erforderlich, da sich die Intensität - und damit zugleich die messbare Druckantwort - auch bei einer reinen Variation des Einfallswinkels verändert.

Mit Hilfe der modellierten Lichtintensitäten kann man erfindungsgemäß die absorbierte Energiedichte auf die einzelnen Schichten des Schichtenstapels aufteilen, anders gesagt, die Beiträge der einzelnen Schichten zur gemessenen Energiedichte ermitteln. Dazu dient die folgende Überlegung:

Die Zunahme der deponierten Energie über ein Tiefenintervall, $\Delta x$, entspricht der Abnahme der eingestrahlten Leistung über dasselbe Intervall multipliziert mit der Dauer des Messlichtpulses, $\Delta t$ (vgl. Fig. 1). Die Messlichtintensität $I(x)$ ist die tiefenabhängige Leistungsflächendichte, und es gilt näherungsweise

$$\frac{\Delta E(x)}{\Delta x} \cong -F \times \Delta t \times \frac{dI(x)}{dx} \qquad (3)$$

wobei auf der rechten Seite zur Vereinfachung vom Differenzenquotienten zum Differentialquotienten übergegangen wurde. Die über die gesamte Eindringtiefe $\mu$ gemittelte absorbierte Energie erhält man durch eine gewichtete Integration

$$\frac{\Delta E}{\mu} = \int_0^\mu \frac{\Delta E(x)}{\Delta x} \times \rho(x) dx \qquad (4)$$

mit Gewichten

$$\rho(x) = \frac{I(x)}{\int_0^\infty I(x')dx'} \tag{5}$$

Die Gewichtsfunktion aus (5) ist fast null für $x > \mu$, also kann die obere Integrationsgrenze in (4) näherungsweise auf $\infty$ gesetzt werden. Setzt man (3) und (5) in (4) ein und dividiert man durch die Fläche F, ergibt sich

$$\frac{\Delta E}{V} = \frac{-\Delta t}{\int_0^\infty I(x')dx'} \times \int_0^\infty \frac{dI(x)}{dx} \times I(x)dx \tag{6}$$

$$\frac{\Delta E}{V} = \frac{\Delta t}{\int_0^\infty I(x')dx'} \times \sum_{i=1}^M \int_{d_{i-1}}^{d_i} \alpha_i \times I^2(x)dx \tag{7}$$

Beim Übergang von (6) zu (7) wird die Probe als Schichtenstapel mit $M$ Schichten der Schichtdicken $d_i$, $i = 1,...,M$ mit der Festlegung $d_0 = 0$ sowie den Absorptionskoeffizienten $\alpha_i$, $i = 1,...,M$ eingeführt. In den einzelnen Schichten folgt die Intensität dem bekannten Exponentialverlauf, und es gilt jeweils $\frac{dI(x)}{dx} = -\alpha_i \times I(x)$.

Die Abhängigkeit vom Einfallswinkel $\theta$ wird in den bisherigen Gleichungen unterdrückt. Nimmt man diese nun explizit hinzu, erhält man als Ergebnis:

$$\frac{\Delta E(\theta)}{F\mu(\theta)} = \frac{\Delta t}{\int_0^\infty I(\theta,x')dx'} \times \sum_{i=1}^M \int_{d_{i-1}/\cos\theta}^{d_i/\cos\theta} \alpha_i \times I^2(\theta,x)dx \tag{8}$$

Gleichung (8) ist ein Bestimmungsgleichungssystem für Parameter, die den Verlauf der Intensität $I(x)$ im Schichtenstapel festlegen, wenn zusätzlich $I_0$ vorbekannt ist. Die linke Seite von (8) repräsentiert gemessene Werte aus den Drucktransienten ausgewertet nach Gleichung (2). Es können Messwerte für eine grundsätzlich beliebige Anzahl $N$ von Einfallswinkeln $\theta$ verwendet werden, um das Gleichungssystem (8) mit linear unabhängigen Gleichungen aufzufüllen.

Es liegt vordergründig beim Anwender der Erfindung, wie er das Modell der inhomogenen Probe gestalten möchte und welche Parameter er als a priori unbekannte Fitparameter auswählt. Beispielsweise könnten die Schichtdicken einer biologischen Probe vorab mit einer Optischen Kohärenztomographie gemessen worden sein. Dann wären die $d_i$, $i = 1, ..., M$ bekannt und keine Fitparameter mehr. Man kann im Regelfall davon ausgehen, dass jede Schicht des Schichtenstapels wenigstens einen unbekannten Fitparameter zugewiesen bekommt. Erfindungsgemäß wird wenigstens einer Schicht ein Absorptionskoeffizient als Fitparameter zugeschrieben.

Vorzugsweise sollte die Anzahl $N$ der in Gleichung (8) in Betracht gezogenen Einfallswinkel ungefähr der Anzahl der unbekannten Fitparameter in Gleichung (8) entsprechen, die unabhängig voneinander variiert werden können. Erfindungsgemäß sollen wenigstens zwei voneinander verschiedene Einfallswinkel verwendet werden.

Es ist im Übrigen auch von der Erfindung umfasst, die beim Aufstellen von Gleichung (2) getroffene Annahme über den Quotienten $\frac{\Gamma'}{C_p}$ zu überprüfen und ggf. noch zu verbessern. Tatsächlich ist die gemessene Druckantwort des Schichtenstapels die Summe der Druckantworten, $\Delta p_i$, der Einzelschichten, und jeder Schicht kann ein eigener Quotient $\frac{\Gamma'_i}{C_p^i}$ zugeordnet werden, der dem $\Delta p_i$ proportional ist. Dies gestattet das Aufsetzen einer Selbstkonsistenzschleife, beispielsweise wie folgt:

a) Messe N Druckantworten für $N$ Einfallswinkel

b) Berechne N Werte für absorbierte Energiedichten nach Gleichung (2)

c) Setze berechnete Energiedichten in Gleichung (8) ein und löse das Gleichungssystem (i.d.R. numerisch) für ein Modell mit M Schichten

d) Weise den M Schichten Quotienten $\frac{\Gamma'_i}{C_p{}^i}$; zu und berechne neuen mittleren Wert $\frac{\Gamma'}{C_p}$

e) Wiederhole Schritte b) bis d), bis sich die Parameter des Modells nicht mehr ändern

Üblicherweise bricht man Selbstkonsistenzschleifen ab, wenn die Änderungen der Parameter zwischen zwei aufeinanderfolgenden Durchläufen unter vorbestimmte Grenzen fallen. Grundsätzlich muss es immer dem Nutzer des Verfahrens vorbehalten bleiben, ein Konsistenzkriterium konkret zu formulieren, da nur er die genaue Natur der Probe und seine Genauigkeitsanforderungen an die zu ermittelnden Parameter kennt.

Zur weiteren Verdeutlichung und zur Erläuterung von Ausgestaltungen dienen die folgenden Figuren. Dabei zeigt:

Fig. 1 einen Plot des typischen Zeitverlaufs eines Messlichtpulses;

Fig. 2 eine Skizze eines Schichtmodells der Probe mit den Parametern, die den einzelnen Schichten zugeordnet werden, und drei Intensitätsverläufen für unterschiedliche Einfallswinkel;

In Fig. 1 ist der Zeitverlauf eines Messlichtpulses exemplarisch dargestellt. Die Intensität steigt während einer kurzen Zeitspanne $\delta t$ von 0 auf ihren Maximalwert $I_0$ an, bleibt dort für eine Zeitspanne $\Delta t \gg \delta t$, und fällt hiernach während $\delta t$ wieder auf 0 zurück. Druckpulse durch Expansion und Kontraktion der Probe werden jeweils während der Anstiegs- oder Abklingdauer der Messlichtpulse ausgelöst und erzeugen während der Zeitintervalle der Länge $\delta t$ die Ausschläge in den messbaren Drucktransienten. Während der Pulsdauer $\Delta t$ findet die Energiedeponierung und Erwärmung der Probe statt. In dieser Phase zeigen die Drucktransienten keine nennenswerte Struktur. Vorzugsweise beträgt die Pulsdauer zwischen 100 und 1000 Nanosekunden, besonders bevorzugt zwischen 200 und 600 Nanosekunden. Dadurch ist sichergestellt, dass der Zeitabstand der beiden Druckpulse so groß ist, dass der erste bereits erfasst worden sein kann - auf jeden Fall den Erwärmungsbereich der Probe längst verlassen hat - bevor der zweite ausgelöst wird. Zugleich ist die Pulsdauer noch zu klein, um zwischen den Auslösezeitpunkten der Druckpulse einen signifikanten Abtransport der deponierten Energie durch Wärmediffusion zu gestatten.

Das Messlicht wird auf eine Messfläche in der Oberfläche der Probe eingestrahlt. Die genaue Definition der Messfläche hinsichtlich Position, Form und Flächeninhalt obliegt dem Nutzer. Aufgrund der vorausgesetzten geringen Eindringtiefe des Messlichts in der Probe ist mit der stärksten Druckantwort auf die photoakustische Anregung direkt an der Messfläche zu rechnen.

Beispielsweise und bevorzugt kann die Einstrahlung des Messlichts durch ein Kontaktprisma erfolgen, das für das Messlicht transparent ist. Das Kontaktprisma weist eine Probenkontaktfläche auf, auf die das Messlicht gerichtet ist, und die sich in mechanischem Kontakt mit der Probe befindet. Druckpulse, die durch das in die Probe eindringende Messlicht erzeugt werden, propagieren bis zur Probenkontaktfläche und können dort abgegriffen werden, beispielsweise durch einen Schallwandler (Piezotransducer), der auf einer der Probenkontaktfläche gegenüberliegenden Detektionsfläche des Kontaktprismas angeordnet ist. Die Druckpulse müssen dafür eine zusätzliche Wegstrecke im Innern des Kontaktprismas durchlaufen und treffen zeitverzögert ein, was aber für die Auswertung nach dem erfindungsgemäßen Verfahren unbedeutend ist. Der Zweck des Zusatzweges besteht allein darin, den Einfall des Messlichts auf die Probe nicht durch den Schallwandler zu behindern. Insofern wird auch die Messung mittels eines Kontaktprismas als Erfassen der Drucktransienten an der Messfläche - hier identisch mit der Probenkontaktfläche - betrachtet.

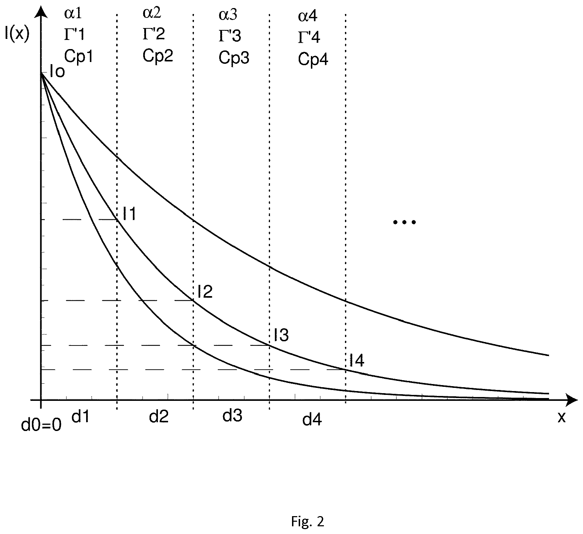

Ein Modell der inhomogenen Probe als Schichtenstapel ist in Fig. 2 dargestellt. Den einzelnen Schichten können Schichtdicken, Absorptionskoeffizienten, Grüneisen-Koeffizienten und Wärmekapazitäten zugeordnet werden. Für die erfindungsgemäße Auswertung benötigt man indes nur den Temperaturanstieg des Grüneisen-koeffizienten im Verhältnis zur Wärmekapazität, also den Quotienten beider. Insofern sind mit dem Verfahren auch nur die Quotienten als Fitparameter verwendbar, wie bereits erläutert, beispielsweise bei der Ausführung von selbstkonsistenten Schleifenberechnungen. Die Einführung von Quotienten aus dem Temperaturanstieg des Grüneisen-Koeffizienten und der Wärmekapazität als Fitparameter für die Einzelschichten des Schichtenstapels ist eine vorteilhafte Ausgestaltung des Verfahrens.

Welche Parameter bekannt sind und welche gefittet werden müssen, hängt grundsätzlich von der Natur der Probe und dem Vorwissen des Nutzers ab. Konkret für lebende biologische Proben, insbesondere für die Glukosebestimmung in lebender Haut, ist jedoch der Absorptionskoeffizient für das Messlicht besonders interessant und üblich aufgrund physiologischer Vorgänge veränderlich und daher unbekannt. Die Erfindung zielt deshalb darauf ab, wenigstens den tiefenaufgelösten Absorptionskoeffizienten, $\alpha(x)$, der inhomogenen Probe aus winkelabhängig erfassten Drucktransienten zu bestimmen. Dies erfolgt näherungsweise durch Identifizieren von $\alpha(x)$ mit schichtweise konstanten Parametern

$\alpha_i, i$ = 1, ..., $M$ eines Schichtenstapel-Modells der Probe, wobei wenigstens ein $\alpha_i$ a priori unbekannt ist und durch eine Fitprozedur bestimmt wird. In der Regel sind alle $\alpha_i$ unbekannt und dienen als Fitparameter der Fitprozedur.

Die Schichtdicken in Fig. 2 sollen vorzugsweise aus dem Bereich 500 Nanometer bis 100 Mikrometer ausgewählt sein. Wesentlich dünnere Schichten als 500 Nanometer werden kaum ausreichende lineare Absorption zeigen und somit keine nennenswerte Energiedeponierung. Es ist zweckmäßiger, solche dünnen Schichten im Verbund mit einer dickeren Nachbarschicht zu modellieren. Das erfindungsgemäße Verfahren umfasst jedoch ausdrücklich auch den Fall des - nahezu trivialen - Modells mit nur genau einer Schicht. Denn auch in diesem Fall wird hier vorgeschlagen, wenigstens zwei Messungen der Drucktransienten, mithin zwei Messungen der deponierten Energiedichte, unter wenigstens zwei verschiedenen Einfallswinkeln vorzunehmen, um die Schichtparameter zu ermitteln. Die erfindungsgemäße Fitprozedur für die Fitparameter kann dabei dann natürlich recht simpel ausfallen.

Anhand der drei Intensitätskurven in Fig. 2 kann die Erfindungsidee noch einmal in anderen Worten verdeutlicht werden. Die obere Kurve kennzeichnet beispielsweise den Intensitätsverlauf des Messlichts in der Probe, wenn das Messlicht senkrecht auf die Messfläche einfällt ($\theta$ = 0°). Das Messlicht dringt am weitesten ein und verliert in den Einzelschichten am wenigsten Leistung, weil es jede der Schichten auf dem kürzesten Weg durchquert. Die zweite Kurve von oben repräsentiert die Intensität bei einem Einfallswinkel $\theta = \varphi > 0°$ und die unterste Kurve beschreibt den Intensitätsverlauf für den Einfallswinkel $\theta = \varphi' > \varphi$. Jede der Einzelschichten wird mit wachsendem Einfallswinkel auf einem längeren Weg durchquert, so dass die Schichten während der Pulsdauer des Messlichtpulses umso mehr Energiedichte akkumulieren, je näher sie sich an der Messfläche befinden. Tiefere Schichten tragen für große Einfallswinkel immer weniger bis gar nichts mehr bei. In der Zusammenschau der winkelabhängigen Drucksignale lässt sich auf die Abfolge und die Eigenschaften der Schichten zurückschließen.

Die Angabe von Zwischenwertintensitäten $I_1, I_2, I_3, ...$ an der mittleren Kurve soll darauf hinweisen, dass die Integrale in Gleichung (8) alle analytisch lösbar sind und dabei auf eben diese Zwischenwerte führen. Es kann hilfreich sein, diese Zwischenwerte als zusätzliche Fitparameter einzuführen und das Gleichungssystem mit einer größeren Zahl an Einfallswinkeln zu lösen. Allerdings hängen die Zwischenwerte von den Absorptionskoeffizienten und Dicken der Schichten direkt ab, so dass man dann noch eine weitere Konsistenzkontrolle durchzuführen hat.

Eine vorteilhafte Möglichkeit, die Fitprozedur auch für Schichtmodelle mit einer Mehrzahl von Einzelschichten und sogar mehreren Fitparametern pro Schicht schnell und effizient auszuführen, bietet sich durch ein geschicktes Verwenden der winkelabhängig gemessenen Drucktransienten bzw. absorbierten Energiedichten an. Wenn man nämlich die Messwerte nach Einfallswinkeln ordnet und zunächst nur Teilmengen der Messwerte für Einfallswinkel $\theta > \theta_0 \gg 0°$ in das Gleichungssystem (8) einfließen lässt - also nicht das vollständige System auswertet -, dann kann man bereits zu verlässlichen Ergebnissen für die Fitparameter der äußersten Schicht(en) gelangen. Die benutzten Messwerte enthalten eben tatsächlich keine Beiträge tieferer Schichten, d.h. dieses Vorgehen stellt keine Näherung dar, sondern umfasst lediglich eine Annahme über die Eindringtiefe des Messlichts unter den selektierten Einfallswinkeln.

Sollte die Annahme grob falsch getroffen werden, d.h. sollte man für die gewählte Teilmenge von Messwerten zu wenige Schichten im Modell vorsehen, dann erhält man zunächst fehlerhafte Fitparameter, die sich bei späterer Hinzunahme von mehr Schichten und unter Verwendung einer größeren Teilmenge der Messwerte wieder in Richtung auf korrekte Werte bewegen werden. Schlimmstenfalls vergeudet man Rechenzeit, erhält aber kein falsches Ergebnis.

Wenn man aber korrekte Annahmen macht, kann sich die Ausführung der Fitprozedur erheblich beschleunigen, weil bevorzugt die Fitparameter des Schichtenstapels für Schichten ausgehend von der äußersten Schicht sukzessiv gefittet werden, wobei das Erfüllen des vorbestimmten Konsistenzkriteriums beginnend mit dem größten Einfallswinkel des Messlichts für sukzessiv kleinere Einfallswinkel wiederholt überprüft wird. Dadurch dass man die Parameter der äußeren Schichten zuerst und die tieferliegenden nach und nach fixiert, kann man Rechenaufwand in den nachfolgenden Schritten des Fitverfahrens einsparen, vor allem wenn man pro Schicht mehrere Materialgrößen als Fitparameter ermitteln will.

Es ist hier zu erwähnen, dass man korrekte Annahmen im Sinne des zuvor Gesagten insbesondere dann machen kann, wenn man eine sich wiederholende Messaufgabe an immer derselben Probe zu lösen hat, wo man aus vorangegangenen Messungen bereits über eine fundierte Historie der möglichen Variationen der Fitparameter verfügt. Dies ist ganz besonders bei wiederholten Glukosemessungen an einem Probanden der Fall.

Wie bereits ausgeführt, ist das erfindungsgemäße Verfahren sehr gut zur Messung an lebenden biologischen Proben, insbesondere an lebenden Hautproben, geeignet. Ein Ziel ist die nichtinvasive Blutglukosebestimmung in vivo an einem menschlichen Körperteil. Für die Zielsetzung ist konkret die Wahl von mittleren Infrarot (MIR) Wellenlängen für das Messlicht besonders geeignet. Vorzugsweise weist das Messlicht Wellenlängen aus dem Intervall von 1 bis 20 Mikrometer, besonders bevorzugt von 6 bis 12 Mikrometer auf.

Es ist offenkundig, dass man die Wellenlänge des Messlichts während der Untersuchung einer einzigen Probe verändern kann. Beispielsweise kann man für eine erste Wellenlänge eine Sequenz von Einfallswinkeln durchlaufen und erste winkelabhängige Drucktransienten aus der Probe erfassen, um danach für eine zweite Wellenlänge dieselbe Sequenz von Einfallswinkeln zur Messung einer zweiten Sequenz von Drucktransienten zu verwenden. Dieses Vorgehen ist insbesondere sinnvoll zur erfindungsgemäßen Bestimmung von Absorptionskoeffizienten, da diese selbst üblich von der Wellenlänge abhängen. Gerade anhand der Wellenlängenabhängigkeit der Absorption erfolgt die spektrale Identifikation gesuchter Substanzen.

Eine vorteilhafte Ausgestaltung der Erfindung wird jedoch darin gesehen, dass die Wellenlänge des Messlichts für vorbestimmte Einfallswinkel über ein vom Einfallswinkel abhängiges Intervall variiert wird. Auch hier liegt der Hauptvorteil in einer Beschleunigung der Untersuchung. Wenn nämlich im Beispiel der Glukosebestimmung an der lebenden Haut die Drucktransienten für große Einfallswinkel - streifender Einfall - gemessen und daraus vorzugsweise Werte der wellenlängenabhängigen Absorptionskoeffizienten für epidermale Schichten, insbesondere für das Stratum Corneum und das Stratum Spinosum, bestimmt werden, dann sind diese Messwerte zunächst nur für das außen liegende Stratum Corneum aussagekräftig. Hierbei weiß man aber aus Voruntersuchungen, dass Glukose kein wesentlicher Bestandteil des Stratum Corneum ist. Es ist daher nicht nötig, eine Mehrzahl von Wellenlänge einzustrahlen, die besonders stark von Glukose absorbiert werden, um einen ohnehin nur schwach veränderlichen Absorptionskoeffizienten in der äußersten Schicht zu verifizieren. Erst wenn das Messlicht auch das Stratum Spinosum erreichen kann, sind Glukosesensitive Wellenlängen sinnvoll und wichtig zur quantitativen Bestimmung der Absorption, damit eine realistische Glukose-Konzentration ermittelt werden kann. Von daher reicht es aus, solche Wellenlängen erst bei kleineren Einfallswinkeln des Messlichts hinzuzuschalten.

Als abschließende Bemerkung soll darauf hingewiesen werden, dass die Veränderung des Einfallswinkels bei der Beleuchtung der Messfläche mit Messlicht den unerwünschten Nebeneffekt haben kann, dass die tatsächlich in die Probe eingestrahlte Intensität variiert, weil ein ebenfalls variabler Lichtanteil von der Probe reflektiert wird. Die reflektierte Intensität kann man allerdings z.B. direkt mit einem Photodetektor messen. Hierdurch ist ohne weiteres ein numerischer Korrekturfaktor für die Intensitäten in den Gleichungen (2) und (8) ermittelbar. Es kann jedoch von Vorteil sein, stattdessen lieber die tatsächlich eintretende Messlichtintensität konstant zu halten, weil ansonsten mit erhöhtem Rauschen bei den gemessenen Drucktransienten zu rechnen ist. Zu diesem Zweck wird vorgeschlagen, das Messlicht durch eine elektronisch schaltbare Abschwächungseinrichtung, beispielsweise linear polarisiert durch einen Solenoid und einen Polarisator hindurch, auf die Messfläche einzustrahlen. Durch die Wahl der Bestromung der Spule lässt sich die Polarisationsebene des Messlichts dann so drehen, dass wahlweise mehr oder weniger Messlicht auf die Messfläche gelangt. Es kann damit eine automatisierbare Nachregelung der Messlichtintensität erfolgen, wenn z.B. besagter Photodetektor fortlaufend die reflektierte Intensität misst und an eine Steuereinheit für den Spulenstrom weitermeldet.

## Patentansprüche

1. Photoakustik-Verfahren mit einem Messlicht aufweisend einen vorbestimmten Wellenlängenbereich zur Bestimmung von Eigenschaften einer inhomogenen Probe, wobei die Probe für den vorbestimmten Wellenlängenbereich eine mittlere Absorptionslänge $\mu$ aus dem Intervall 1 - 100 Mikrometer aufweist, umfassend die Schritte:

    a) Einstrahlen von wenigstens einem Messlichtpuls mit vorbestimmter Pulsdauer und vorbestimmter Intensität auf eine Messfläche der Fläche $F$ mit $\sqrt{F} \gg \mu$ in der Oberfläche der inhomogenen Probe;
    b) Detektieren von wenigstens einer Drucktransienten an der Messfläche, wobei die Drucktransiente durch Absorption des wenigstens einen Messlichtpulses in der inhomogenen Probe unter Erzeugen von einer zur Messfläche propagierenden Druckwelle entsteht;
    c) Berechnen eines Wertes für die während der Pulsdauer von der Probe absorbierte Energiedichte aus dem Verlauf der wenigstens einen Drucktransienten am Anfang und am Ende des wenigstens einen Messlichtpulses; **gekennzeichnet durch**
    d) Wiederholen der Schritte a) bis c) für voneinander verschiedene Einfallswinkel des Messlichts gegenüber der Normalen der Messfläche, wobei die in c) bestimmten Energiedichtewerte jeweils mit dem Einfallswinkel indiziert werden;
    e) Modellieren der inhomogenen Probe als Schichtenstapel, wobei jeder Schicht wenigstens eine Schichtdicke und ein Absorptionskoeffizient zugeordnet werden, wobei wenigstens ein Absorptionskoeffizient einer Schicht ein Fitparameter ist;
    f) Ausführen einer Fitprozedur für die Fitparameter des Schichtenstapels, wobei durch Variation der Fitparameter die Aufteilung der mit dem Einfallswinkel indizierten Energiedichtewerte auf Beiträge der einzelnen Schichten variiert wird, bis ein vorbestimmtes Konsistenzkriterium erfüllt ist;
    g) Auslesen der gefitteten Fitparameter als Werte wenigstens für den tiefenaufgelösten Absorptionskoeffizienten der inhomogenen Probe.

2. Photoakustik-Verfahren nach Anspruch 1, **dadurch gekennzeichnet, dass** in Schritt f) jeder Schicht weiterhin ein Quotient aus der Temperaturableitung des Grüneisen-Koeffizient und der Wärmekapazität des Schichtmaterials als Fitparameter zugeordnet werden.

**3.** Photoakustik-Verfahren nach einem der Ansprüche 1 oder 2, **dadurch gekennzeichnet, dass** die Fitparameter des Schichtenstapels für Schichten ausgehend von der äußersten Schicht sukzessiv gefittet werden, wobei das Erfüllen des vorbestimmten Konsistenzkriteriums beginnend mit dem größten Einfallswinkel des Messlichts für sukzessiv kleinere Einfallswinkel wiederholt überprüft wird.

**4.** Photoakustik-Verfahren nach einem der vorangehenden Ansprüche, **dadurch gekennzeichnet, dass** die Pulsdauer der Messlichtpulse auf einen Wert aus dem Intervall von 100 bis 1000 Nanosekunden, bevorzugt aus dem Intervall von 200 bis 600 Nanosekunden, vorbestimmt wird.

**5.** Photoakustik-Verfahren nach einem der vorangehenden Ansprüche, **dadurch gekennzeichnet, dass** der Schichtenstapel Einzelschichten mit Schichtdicken aus dem Intervall von 500 Nanometer bis 100 Mikrometer aufweist.

**6.** Photoakustik-Verfahren nach Anspruch 4, **dadurch gekennzeichnet, dass** die inhomogene Probe eine biologische Probe, vorzugsweise eine lebende Probe, besonders bevorzugt eine lebende Hautprobe, ist.

**7.** Photoakustik-Verfahren nach einem der vorangehenden Ansprüche, **dadurch gekennzeichnet, dass** das Messlicht Wellenlängen aus dem Intervall 1 bis 20 Mikrometer, bevorzugt 6 bis 12 Mikrometer aufweist.

**8.** Photoakustik-Verfahren nach Anspruch 6, **dadurch gekennzeichnet, dass** die Wellenlänge des Messlichts für vorbestimmte Einfallswinkel über ein vom Einfallswinkel abhängiges Intervall variiert wird.

**9.** Photoakustik-Verfahren nach den Ansprüchen 6 bis 8, **dadurch gekennzeichnet, dass** die wellenlängenabhängigen Absorptionskoeffizienten für epidermale Schichten bestimmt werden, insbesondere für das Stratum Corneum und das Stratum Spinosum.

**Claims**

**1.** A photoacoustic method with a measuring light having a predetermined wavelength range for the determination of properties of an inhomogeneous sample, wherein the sample has an average absorption length $\mu$ from the interval 1-100 micrometres for the predetermined wavelength range, comprising the steps:

a) irradiating a measurement area of the area $F$ with $\sqrt{F} \gg \mu$ in the surface of the inhomogeneous sample with at least one measuring light pulse with a predetermined pulse duration and a predetermined intensity;
b) detecting at least one pressure transient at the measurement area, wherein the pressure transient results from the absorption of the at least one measuring light pulse in the inhomogeneous sample which generates a pressure wave propagating to the measurement area;
c) calculating a value for the energy density absorbed by the sample during the pulse duration from the curve of the at least one pressure transient at the beginning and at the end of the at least one measuring light pulse;
**characterized by**
d) repeating steps a) to c) for different angles of incidence of the measuring light with respect to the normal of the measurement area, wherein the energy density values determined in c) are respectively indexed with the angle of incidence;
e) modelling the inhomogeneous sample as a stack of layers, wherein at least a layer thickness and an absorption coefficient are assigned to each layer, wherein at least one absorption coefficient of a layer is a fit parameter;
f) performing a fitting procedure for the fit parameters of the stack of layers, wherein the division of the energy density values indexed with the angle of incidence into contributions of the individual layers is varied by variation of the fit parameters until a predetermined consistency criterion is satisfied;
g) reading out the fitted fit parameters as values at least for the depth-resolved absorption coefficient of the inhomogeneous sample.

**2.** The photoacoustic method according to claim 1, **characterized in that**, in step f), a quotient of the temperature derivation of the Grüneisen coefficient and the thermal capacity of the layer material are assigned as fit parameters to each layer.

**3.** The photoacoustic method according to claim 1 or 2, **characterized in that** the fit parameters of the stack of layers are successively fitted for layers based on the outermost layer, wherein the satisfaction of the predetermined consistency criterion is checked repeatedly, beginning with the largest angle of incidence of the measuring light, for successively smaller angles of incidence.

4. The photoacoustic method according to one of the preceding claims, **characterized in that** the pulse duration of the measuring light pulses is predetermined at a value from the interval of 100 to 1000 nanoseconds, preferably from the interval of 200 to 600 nanoseconds.

5. The photoacoustic method according to one of the preceding claims, **characterized in that** the stack of layers has individual layers with layer thicknesses from the interval of 500 nanometres to 100 micrometres.

6. The photoacoustic method according to claim 4, **characterized in that** the inhomogeneous sample is a biological sample, preferably a living sample, particularly preferably a living skin sample.

7. The photoacoustic method according to one of the preceding claims, **characterized in that** the measuring light has wavelengths from the interval 1 to 20 micrometres, preferably 6 to 12 micrometres.

8. The photoacoustic method according to claim 6, **characterized in that** the wavelength of the measuring light for predetermined angles of incidence is varied over an interval that is dependent on the angle of incidence.

9. The photoacoustic method according to claims 6 to 8, **characterized in that** the wavelength-dependent absorption coefficients are determined for epidermal layers, in particular for the stratum corneum and the stratum spinosum.

**Revendications**

1. Procédé photoacoustique avec une lumière de mesure présentant une gamme de longueurs d'onde prédéfinie, pour déterminer des propriétés d'un échantillon inhomogène, l'échantillon présentant pour la gamme de longueurs d'onde prédéfinie une longueur d'absorption moyenne $\mu$ de l'intervalle de 1 à 100 micromètres, comprenant les étapes suivantes consistant à :

a) irradier au moins une impulsion de lumière de mesure d'une durée d'impulsion prédéfinie et d'une intensité prédéfinie sur une surface de mesure de surface $F$ avec $\sqrt{F} \gg \mu$ dans la surface de l'échantillon inhomogène ;

b) détecter au moins une pression transitoire sur la surface de mesure, la pression transitoire se produisant par absorption de l'au moins une impulsion de lumière de mesure dans l'échantillon inhomogène sous création d'une onde de pression se propageant vers la surface de mesure ;

c) calculer une valeur pour la densité énergétique absorbée par l'échantillon pendant la durée d'impulsion, à partir de la courbe de l'au moins une pression transitoire au début et à la fin de l'au moins une impulsion de lumière de mesure ;

**caractérisé par**

d) la répétition des étapes a) à c) pour des angles d'incidence différents les uns des autres de la lumière de mesure par rapport aux normales de la surface de mesure, les valeurs de densité énergétiques déterminées dans l'étape c) étant indexées respectivement avec l'angle d'incidence ;

e) la modélisation de l'échantillon inhomogène en tant que pile de couches, à chaque couche étant associés au moins une épaisseur de couche et un coefficient d'absorption, au moins un coefficient d'absorption d'une couche étant un paramètre d'ajustement ;

f) la réalisation d'une procédure d'ajustement pour les paramètres d'ajustement de la pile de couches, par variation des paramètres d'ajustement, en faisant varier la répartition des valeurs de densité énergétique indexées avec l'angle d'incidence sur des contributions des couches individuelles, jusqu'à ce qu'un critère de consistance prédéfini soit satisfait ;

g) la lecture des paramètres d'ajustement ajustés en tant que valeurs au moins pour le coefficient d'absorption à résolution en profondeur.

2. Procédé photoacoustique selon la revendication 1, **caractérisé en ce que** dans l'étape f), il est associé par ailleurs à chaque couche en tant que paramètre d'ajustement un quotient de la dérivation de température du coefficient de Grüneisen et de la capacité thermique de la matière des couches.

3. Procédé photoacoustique selon l'une quelconque des revendications 1 ou 2, **caractérisé en ce que** les paramètres d'ajustement de la pile de couches sont ajustés successivement pour des couches à partir de la couche la plus à l'extérieur, la satisfaction du critère de consistance prédéfini étant vérifiée de manière répétée en commençant par le plus grand angle d'incidence de la lumière de mesure pour des angles d'incidence successivement plus petits.

4. Procédé photoacoustique selon l'une quelconque des revendications précédentes, **caractérisé en ce que** la durée d'impulsion des impulsions de lumière de mesure est prédéfinie à une valeur de l'intervalle de 100 à 1000 nanosecondes, de préférence de l'intervalle de 200 à 600 nanosecondes.

5. Procédé photoacoustique selon l'une quelconque des revendications précédentes, **caractérisé en ce que** la pile de couches comporte des couches individuelles ayant des épaisseurs de couches de l'intervalle de 500 nanomètres à 100 micromètres.

6. Procédé photoacoustique selon la revendication 4, **caractérisé en ce que** l'échantillon inhomogène est un échantillon biologique, de préférence un échantillon vivant, de manière particulièrement préférentielle, un échantillon cutané vivant.

7. Procédé photoacoustique selon l'une quelconque des revendications précédentes, **caractérisé en ce que** la lumière de mesure présente des longueurs d'onde de l'intervalle de 1 à 20 micromètres, de préférence de 6 à 12 micromètres.

8. Procédé photoacoustique selon la revendication 6, **caractérisé en ce qu'**on fait varier la longueur d'onde de la lumière de mesure pour des angles d'incidence prédéfinis sur un intervalle dépendant de l'angle d'incidence.

9. Procédé photoacoustique selon les revendications 6 à 8, **caractérisé en ce qu'**on détermine les coefficients d'absorption dépendant des longueurs d'onde pour des couches épidermiques, notamment pour la stratum corneum et la stratum spinosum.

Fig. 1

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **FEI GAO et al.** Single laser pulse generated dual photoacoustic signals for differential contrast photoacoustic imaging. *Scientific Reports,* 2017, vol. 7, 626

- **VON KOTTMANN et al.** Glucose sensing in human epidermis using midinfrared photoacoustic detection. *BIOMEDICAL OPTICS EXPRESS,* vol. 3 (4), 667